# EUROPEAN PATENT SPECIFICATION

(11) **EP 1 818 045 B1**
(45) Date of publication and mention of the grant of the patent: **17.10.2007**
(21) Application number: 06425071.5
(22) Date of filing: 10.02.2006
(51) Int. Cl.: A61K 9/06, A61P 29/00

(54) **Antiphlogistic and analgesic compound for topical use in an area of an animal locomotive system**
Zusammensetzung enthaltend antiphlogistisch und analgetisch wirkende Bestandteile zur topischen Anwendung am tierischen Bewegungsapparat
Composition antiphlogistic et analgetic pour utilisation topique sur le systeme locomotrice d'une animal

(43) Date of publication of application: 15.08.2007
(73) Proprietor: Lombardo, Vincenzo Massimo, 20124 Milano (IT); Molinari, Alberto Antonio, 20144 Milano (IT); Ferraiolo, Ruggero, 20124 Milano (IT)
(72) Inventor: Lombardo, Vicenzo Massimo, 20124 Milano (IT); Molinari, Alberto Antonio, 20144 Milano (IT)
(74) Representative: Ferraiolo, Ruggero

(56) References cited:
- BR-A- 9 404 286
- US-A- 4 973 473
- US-A- 5 085 870
- US-A- 5 976 547

## Description

The present invention refers to an antiphlogistic and analgesic composition for use on the epidermis site where pains are felt from muscles, articulations, tendons and ligaments caused by pathologies, contusions and trauma.

The prior art comprises a number of compositions in the form of ointment, cream, spray or foam which are able to assuage the above specified pains and treat their cause in a notable or little extent. As examples, the commercial products are cited that are sold under the names of Voltaren ®, Algesal ®, Lasonil ®, Artrosilene ® wherein the active ingredients are, respectively, diclofenac diethyl ammonium, diethyl amine salicylate , ketoprofene, lysine salt ketoprofene whereas there is a number of other products used to the same purposes that employ other active ingredients comprised in the pharmacopeia relevant to said purposes. Also document JP 11 279 065 is known that discloses an anti-inflammatory product in the form of topical solution composed by two water soluble anti-inflammatory agents, one of the steroidal type and the other of non-steroidal type, and by hyaluronic acid.

The efficacy of the analgesic and anti-inflammatory effect of the known compositions is affected by the fact that such compositions comprise in most cases an active ingredient only which is not well vehiculated into the suffering body region. Consequently, the efficacy of said compositions may be of short time and not so extended in the concerned region. Moreover, the known compositions may cause undesired effects due to light or notable hyperaesthesia against one or more components and to allergic reactions .

The invented compound obviates the above mentioned limitations and drawbacks and offers advantages that will be described fartheron.

The invented composition comprises as active ingredients a salt of hyaluronic acid, an arnica extract and a non steroidal anti-inflammatory agent (FANS - as for instance methyl salicylate, diclofenac, lysine salt ketoprofene) soluble in a gel and, as vehicle of the active ingredients, hydrogel of the type used in medicine for applying ultrasounds and electric transmissions, type of gel which proved to give the composition the best physical properties and that will be referred to as - hydrogel - in the following.

The hyaluronic acid as anti-inflammatory and chemotactic agent is known for a long time and is generally and successfully used into injectable solutions for treating osteoarthrosis, gonarthrosis, wounds and burns, in the beauty surgery and culture and also as a component for ophthalmic use. In the invented composition the hyarulonic acid is preferably used as sodium hyaluronate, dissolved in just necessary purified water before preparing the composition.

The efficacy is known for a long time of : arnica in the treatment of consequences of trauma and of symptoms similar with "post-trauma"; FANS in the treatment of pains and as antiphlogistic agent ; hydrogel as vehicle of medicaments applied to the epidermis.

The combination in the invented composition of said three active ingredients and the presence of a hydrogel, all the four in the proposed proportions, leads to a fluid and rubbable product which is absorbed rapidly by the epidermis and is of high efficacy in assuaging pains of articular and osteomuscular origin, in the reduction of possible inflammation and in the absorption of possible serous or blood extravasation caused by trauma or contusion.

Such an efficacy is substantially due to the anti-inflammatory and chemotactic properties as well as to the notable hydrophilia of the hyaluronic acid. The latter makes pores of the basal membrane dilate and, supported by the high vehiculation favoured by the hydrogel, causes itself and the other two antiphlogistic and analgesic components to rapidly penetrate into the suffering region Moreover, undesirable effects and allergic reactions of this composition have not been recorded in case of long-term use.

According to first formulae of this composition that proved to be of considerable efficacy through tests carried out on many persons, a hundred grams of the composition comprise weight :
- sodium hyaluronate from 0.01% to 3.00 %,
- arnica glycolic extract from 0.5% to 7.0%,
- any hydrogel-soluble non steroidal anti-inflammatory agent from 2% to 10%,
- hydrogel from 70% to 95%,
- purified water 0.2 %,

As it is known, there is a number of non steroidal anti-inflammatory agents and the inventors proved that each of them, provided is soluble in hydrogel, may be used in this composition in a proportion from 2% to 10% in order to provide the composition with the desired efficacy. It has been proved that the most afficacious anti-inflammatory agent in the invented composition is methyl salicylate.

According to a formula of this composition that proved to be the most efficacious through tests carried out on a great number of persons and in a number of percentage alternatives for the four components, the following formula in weight reaches the desired purposes :
- sodium hyaluronate 0.2 %,
- purified water 0.2 %,
- arnica glycolic extract 2 %,
- methyl salicylate 5%,
- hydrogel 92.6 %.

## Claims

1. Antiphlogistic and analgesic composition for use in a region of an animal locomotive system comprising as active ingredients a non steroidal anti-inflammatory agent and hyaluronic acid and arnica mixed in a product adapted to vehiculate the active ingredients **characterized in that** the composition is a mixture of a salt of hyaluronic acid, an arnica glycolic extract and a hydrogel-soluble non steroidal anti-inflammatory agent and hydrogel as vehicle of the active ingredients.

2. Composition according to claim 1 **characterized in that** it comprises by weight
- sodium hyaluronate from 0.01 % to 3.0 %,
- arnica glycolic extract from 0.5 % to 7.0 %,
- any hydrogel-soluble non steroidal anti- inflammatory agent from 2% to 10%,
- hydrogel from 70% to 95%,
- purified water 0.2%.

3. Composition according to claims 1 and 2 **characterized in that** the hydrogel-soluble non steroidal anti- inflammatory agent is methyl salicylate.

4. Antiphlogistic and analgesic composition for use in a region of an animal locomotive system comprising as active ingredients a non steroidal anti-inflammatory agent and hyaluronic acid and arnica mixed in a product adapted to vehiculate the active ingredients **characterized in that** the composition consists by weight of
- sodium hyaluronate 0.2%,
- purified water 0.2%,
- arnica glycolic extract 2%,
- methyl salicylate 5%,
- hydrogel 92,6%.

## Patentansprüche

1. Antiphlogistische und analgetische Zusammensetzung zur Verwendung in einem Bereich eines tierischen Bewegungsapparats, die als Wirkstoffe ein nichtsteroidales antiphlogistisches Mittel und Hyaluronsäure und Arnika in einem Produkt gemischt, das als Träger der Wirkstoffe geeignet ist, umfasst, **dadurch gekennzeichnet, dass** die Zusammensetzung eine Mischung aus einem Salz von Hyaluronsäure, einem glykolischen Arnikaextrakt und einem hydrogellöslichen nichtsteroidalen antiphlogistischen Mittel und Hydrogel als Träger der Wirkstoffe ist.

2. Zusammensetzung gemäß Anspruch 1, **dadurch gekennzeichnet, dass** sie nach Gewicht umfasst
- Natriumhyaluronat von 0,01 % bis 3,0 %,
- glykolischen Arnikaextrakt von 0,5 % bis 7,0 %,
- ein beliebiges hydrogellösliches nichtsteroidales antiphlogistisches Mittel von 2 % bis 10 %,
- Hydrogel von 70 % bis 95 %,
- gereinigtes Wasser 0,2 %.

3. Zusammensetzung gemäß den Ansprüchen 1 und 2, **dadurch gekennzeichnet, dass** das hydrogellösliche nichtsteroidale antiphlogistische Mittel Methylsalicylat ist.

4. Antiphlogistische und analgetische Zusammensetzung zur Verwendung in einem Bereich eines tierischen Bewegungsapparats, die als Wirkstoffe ein nichtsteroidales antiphlogistisches Mittel und Hyaluronsäure und Arnika in einem Produkt gemischt, das als Träger der Wirkstoffe geeignet ist, umfasst, **dadurch gekennzeichnet, dass** die Zusammensetzung nach dem Gewicht besteht aus
- Natriumhyaluronat 0,2 %,
- gereinigtes Wasser 0,2 %,
- glykolischen Arnikaextrakt 2 %,
- Methylsalicylat 5 %,
- Hydrogel 92,6 %.

## Revendications

1. Composition anti-inflammatoire et analgésique pour utilisation topique sur le système locomoteur d'un animal, comprenant comme principes actifs un agent anti-inflammatoire non stéroïde et de l'acide hyaluronique ainsi que de l'arnica, mélangés dans un produit adapté pour véhiculer les principes actifs,
**caractérisée en ce que**
la composition est un mélange d'un sel d'acide hyaluronique, d'un extrait glycolique d'arnica et d'un agent anti-inflammatoire non stéroïde soluble dans un hydrogel, et d'hydrogel en tant que véhicule des principes actifs.

2. Composition selon la revendication 1,
**caractérisée en ce qu'**
elle contient en poids :
- de 0,01 % à 3,0 % de hyaluronate de sodium,
- de 0,5 % à 7,0 % d'extrait glycolique d'arnica,
- de 2 % à 10 % d'un agent anti-inflammatoire non stéroïde soluble dans un hydrogel,
- de 70 % à 95 % d'hydrogel,
- 0,2 % d'eau purifiée.

3. Composition selon les revendications 1 et 2,
**caractérisée en ce que**
l'agent anti-inflammatoire non stéroïde soluble dans un hydrogel est du salicylate de méthyle.

4. Composition anti-inflammatoire et analgésique pour utilisation topique sur le système locomoteur d'un animal, comprenant comme principes actifs un agent anti-inflammatoire non stéroïde et de l'acide hyaluronique ainsi que de l'arnica, mélangés dans un produit adapté pour véhiculer les principes actifs,
**caractérisée en ce que**
la composition consiste en poids de :
- 0,2 % de hyaluronate de sodium,
- 0,2 % d'eau purifiée,
- 2 % d'extrait glycolique d'arnica,
- 5 % de salicylate de méthyle,
- 92,6 % d'hydrogel
